# EUROPÄISCHE PATENTSCHRIFT

(11) **EP 3 324 936 B1**
(45) Veröffentlichungstag und Bekanntmachung des Hinweises auf die Patenterteilung: **28.09.2022**
(21) Anmeldenummer: 16742280.7
(22) Anmeldetag: 21.07.2016
(51) Int. Cl.: A61K 9/08, A61K 31/69

(54) **VERFAHREN ZUR HERSTELLUNG EINER BORTEZOMIBESTER-LÖSUNG**
PROCESS FOR THE PREPARATION OF A BORTEZOMIB ESTER SOLUTION
PROCÉDÉ DE PRÉPARATION D'UNE SOLUTION D'ESTER DE BORTEZOMIB

(30) Priorität: 22.07.2015 EP 15002164
(43) Veröffentlichungstag der Anmeldung: 30.05.2018
(60) Teilanmeldung: 22190533.4
(73) Patentinhaber: STADA Arzneimittel AG, 61118 Bad Vilbel (DE)
(72) Erfinder: SCHRIDDE, Edgar, 30855 Langenhagen (DE)
(74) Vertreter: Kernebeck, Thomas
(86) Internationale Anmeldenummer: PCT/EP2016/067416
(87) Internationale Veröffentlichungsnummer: WO 2017/013209

(56) Entgegenhaltungen:
- EP-A1- 2 644 189
- WO-A1-2015/025000
- WO-A2-2013/128419
- CN-A- 103 070 835
- I Butler ET AL: "Removal of dissolved oxygen from water: A comparison of four common techniques", TALANTA, vol. 41, no. 2, 1 February 1994 (1994-02-01), pages 211-215, XP055449254, NL ISSN: 0039-9140, DOI: 10.1016/0039-9140(94)80110-X

## Beschreibung

Die vorliegende Erfindung betrifft ein Verfahren zur Herstellung einer gebrauchsfertigen flüssigen pharmazeutischen Bortezomibester-Lösung.

Bortezomib ist der internationale Freiname (INN (*international nonproprietary name*)) für (1R)-3-Methyl-1-[{(2S)-3-phenyl-2-[(2-pyrazinylcarbonyl)-amino]propanoyl)amino]butylborsäure, die die folgende Strukturformel aufweist:

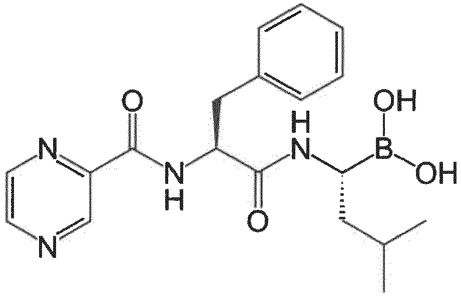

Bortezomib ist ein Arzneistoff aus der Gruppe der Proteasom-Inhibitoren, der für die Mono- und Kombinationstherapie zur Behandlung des multiplen Myeloms und des Mantelzelllymphoms zugelassen ist. Die Wirkung von Bortezomib beruht dabei auf der selektiven Hemmung der Chymotrypsin-artigen Aktivität des 26S-Proteasoms (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 947; ISBN 978-3-8047-1952-1).

Bortezomib wird unter der Markenbezeichnung Velcade^{®} in Form eines lyophilisierten Pulvers in den Wirkstärken 1 mg und 3,5 mg Bortezomib vertrieben. Bortezomib ist in Velcade^{®} in Form eines Mannitol-Boronsäureesters enthalten, der nach Rekonstitution die pharmazeutisch aktive Komponente Bortezomib *in vivo* relativ gut freisetzen soll. Für die intravenöse Verabreichung wird das in einer Durchstechflasche (Vial) enthaltene sterile Pulver mit einer 0,9 %igen Kochsalzlösung rekonstituiert, wobei eine Lösung mit 1 mg/ml Bortezomib zur intravenösen oder 2,5 mg/ml Bortezomib zur subkutanen Applikation hergestellt wird. Die Lagerstabilität einer ungeöffneten Durchstechflasche Velcade^{®} wird bei einer Temperatur bis zu 30 °C mit 3 Jahren angegeben, die einer durch Rekonstitution von Velcade^{®} erhaltenen Bortezomib-Lösung bei 25 °C hingegen nur mit 8 Stunden.

WO 2015/025000 A1 offenbart eine lyophilisierte pharmazeutische Zusammensetzung, umfassend Bortezomib in Mischung mit Natriumgluconat, ein Verfahren zu deren Herstellung sowie eine flüssige pharmazeutische Zusammensetzung, umfassend eine Lösung einer Zusammensetzung, umfassend Bortezomib und Natriumgluconat in einem zur parenteralen Verabreichung geeigneten Verdünnungsmittel und ein Verfahren zur Herstellung der flüssigen Zusammensetzung umfassend die Rekonstitution der lyophilisierten Zusammensetzung mit einem zur parenteralen Verabreichung geeigneten Verdünnungsmittel.

EP 2 644 189 A1 offenbart eine lagerbeständige flüssige pharmazeutische Zusammensetzung, umfassend Bortezomib in einer therapeutisch wirksamen Menge, wobei die Zusammensetzung Folgendes umfasst: eine einphasige flüssige Formulierung, umfassend ein im Wesentlichen nicht wässriges Lösungsmittel-System, das zur Injektion geeignet ist, einen wässrigen Acetatpuffer und Bortezomib, wobei Bortezomib in der Formulierung in einer therapeutisch wirksamen Konzentration vorhanden ist, wobei das Lösungsmittel-System als vorherrschende Komponente Propylenglykol umfasst und wobei der Puffer einen pH-Wert von 3 aufweist, und wobei das Lösungsmittel-System, der Puffer und der pH-Wert so gewählt werden, um wirksam die Bildung von mindestens einem aus einem Amidabbauprodukt, einem ersten Carbinolamidabbauprodukt und einem zweiten Carbinolamidabbauprodukt zu unterdrücken, wenn die flüssige Formulierung unter Lagerungsbedingungen gelagert wird.

WO 2013/128419 A2 offenbart pharmazeutische Zusammensetzungen umfassend Bortezomib, Tromethamin und eine organische Carbonsäure, wobei die Zusammensetzung einen pH-Wert von ungefähr 3 bis 6 aufweist.

Im Hinblick auf eine sicherere Handhabung des Zytostatikums Bortezomib wäre es wünschenswert, wenn man Bortezomib in Form einer gebrauchsfertigen Lösung am Markt anbieten könnte. Zwar ist Bortezomib-Mannitolester in fester Formulierung verhältnismäßig stabil, nicht jedoch in wässriger Formulierung, da freies Bortezomib in wässriger Lösung sehr oxidationsempfindlich ist und Bortezomib-Mannitolester in Lösung im Gleichgewicht mit seinen Hydrolyseprodukten freies Bortezomib und Mannitol vorliegt.

Entsprechend schlägt WO 2011/116286 A2 vor, eine gebrauchsfertige Bortezomib-Lösung auf Basis des organischen Lösungsmittels Propylenglykol herzustellen. Es wird behauptet, dass eine derartig hergestellte Lösung die an eine gebrauchsfertige Lösung zu stellenden Anforderungen hinsichtlich Stabilität und damit Lagerfähigkeit erfüllt. Nachteilig an besagt hergestellter Lösung ist allerdings, dass Propylenglykol starke Reizungen verursachen sowie zu Leberanomalien und Nierenschäden führen kann.

Aufgabe der vorliegenden Erfindung ist es daher, ein Verfahren zur Herstellung einer gebrauchsfertigen flüssigen pharmazeutischen Bortezomibester-Lösung bereitzustellen, wobei die Bortezomibester-Lösung eine verbesserte physiologische Toleranz aufweist.

Diese Aufgabe wird gelöst durch ein Verfahren zur Herstellung einer gebrauchsfertigen flüssigen pharmazeutischen Bortezomibester-Lösung, umfassend die Schritte des
a) Bereitstellens eines wässrigen Lösungsmittels;
b) Lösens einer Bortezomibester-Bildungskomponente in dem Lösungsmittel, wobei als Bortezomibester-Bildungskomponente Mannitol eingesetzt wird;
c) Lösens von Bortezomib oder einem pharmazeutisch annehmbaren Salz davon in dem Lösungsmittel;
d) Verminderns des Sauerstoffgehaltes des Lösungsmittels auf einen Wert von kleiner als 0,5 mg/l.

Überraschenderweise wurde festgestellt, dass eine gebrauchsfertige flüssige pharmazeutische Bortezomibester-Lösung auf der Basis eines wässrigen Lösungsmittels hergestellt werden kann, indem i) eine Bortezomibester-Bildungskomponente in dem Lösungsmittel gelöst wird, wobei als Bortezomibester-Bildungskomponente Mannitol eingesetzt wird ii) Bortezomib oder ein pharmazeutisch annehmbares Salz davon in dem Lösungsmittel gelöst wird und iii) der Sauerstoffgehalt des Lösungsmittels auf einen Wert von kleiner als 0,5 mg/l vermindert wird. Die erfindungsgemäß hergestellte Lösung zeichnet sich aufgrund ihres Wassergehaltes durch eine verbesserte physiologische Toleranz aus.

Alternativ zu den Schritten b) und c) des erfindungsgemäßen Verfahrens kann natürlich auch in einem einzelnen Verfahrensschritt der fertige Bortezomibester, beispielsweise in fester oder flüssiger Form, in dem Lösungsmittel gelöst werden. Bortezomibester sowie Verfahren zu deren Herstellung sind beispielsweise in WO 96/13266 und EP 1 355 910 offenbart.

Die erfindungsgemäß hergestellte Lösung weist ferner den Vorteil auf, dass sie stabil ist.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Die erfindungsgemäß hergestellte Lösung weist bei einer Temperatur von 2 bis 8 °C eine Stabilität (oder anders gesagt Lagerfähigkeit oder Laufzeit) von zumindest 3 Monaten auf, bevorzugt von zumindest 6 Monaten, weiter bevorzugt von zumindest 12 Monaten, noch weiter bevorzugt von zumindest 24 Monaten und am meisten bevorzugt von zumindest 36 Monaten, wobei der Bortezomibgehalt bis zum Ende der Laufzeit 90 %, bevorzugt 95 %, des ursprünglich eingesetzten Bortezomibs nicht unterschreitet.

Die erfindungsgemäß hergestellte Lösung weist ferner erfindungsgemäß bevorzugt bei einer Temperatur von 2 bis 8 °C eine Stabilität (oder anders gesagt Lagerfähigkeit oder Laufzeit) von 3 Monaten auf, mehr bevorzugt eine Stabilität von 6 Monaten, mehr bevorzugt eine Stabilität von 9 Monaten, mehr bevorzugt eine Stabilität von 12 Monaten, mehr bevorzugt eine Stabilität von 15 Monaten, mehr bevorzugt eine Stabilität von 18 Monaten, mehr bevorzugt eine Stabilität von 21 Monaten, mehr bevorzugt eine Stabilität von 24 Monaten, mehr bevorzugt eine Stabilität von 27 Monaten, mehr bevorzugt eine Stabilität von 30 Monaten, mehr bevorzugt eine Stabilität von 33 Monaten und noch mehr bevorzugt eine Stabilität von 36 Monaten, wobei der Bortezomibgehalt bis zum Ende der Laufzeit 90 % des ursprünglich eingesetzten Bortezomibs nicht unterschreitet.

Die erfindungsgemäß hergestellte Lösung weist ferner erfindungsgemäß bevorzugt bei einer Temperatur von 2 bis 8 °C eine Stabilität (oder anders gesagt Lagerfähigkeit oder Laufzeit) von 3 Monaten bis zu 6 Monaten auf, mehr bevorzugt eine Stabilität von 3 bis zu 9 Monaten, mehr bevorzugt eine Stabilität von 3 bis zu 12 Monaten, mehr bevorzugt eine Stabilität von 3 bis zu 15 Monaten, mehr bevorzugt eine Stabilität von 3 bis zu 18 Monaten, mehr bevorzugt eine Stabilität von 3 bis zu 21 Monaten, mehr bevorzugt eine Stabilität von 3 bis zu 24 Monaten, mehr bevorzugt eine Stabilität von 3 bis zu 27 Monaten, mehr bevorzugt eine Stabilität von 3 bis zu 30 Monaten, mehr bevorzugt eine Stabilität von 3 bis zu 33 Monaten und noch mehr bevorzugt eine Stabilität von 3 bis zu 36 Monaten, wobei der Bortezomibgehalt bis zum Ende der Laufzeit 90 % des ursprünglich eingesetzten Bortezomibs nicht unterschreitet.

Die erfindungsgemäß hergestellte Lösung weist ferner erfindungsgemäß bevorzugt bei einer Temperatur von 2 bis 8 °C eine Stabilität (oder anders gesagt Lagerfähigkeit oder Laufzeit) von 3 Monaten auf, mehr bevorzugt eine Stabilität von 6 Monaten, mehr bevorzugt eine Stabilität von 9 Monaten, mehr bevorzugt eine Stabilität von 12 Monaten, mehr bevorzugt eine Stabilität von 15 Monaten, mehr bevorzugt eine Stabilität von 18 Monaten, mehr bevorzugt eine Stabilität von 21 Monaten, mehr bevorzugt eine Stabilität von 24 Monaten, mehr bevorzugt eine Stabilität von 27 Monaten, mehr bevorzugt eine Stabilität von 30 Monaten, mehr bevorzugt eine Stabilität von 33 Monaten und noch mehr bevorzugt eine Stabilität von 36 Monaten, wobei der Bortezomibgehalt bis zum Ende der Laufzeit 95 % des ursprünglich eingesetzten Bortezomibs nicht unterschreitet.

Die erfindungsgemäß hergestellte Lösung weist ferner erfindungsgemäß bevorzugt bei einer Temperatur von 2 bis 8 °C eine Stabilität (oder anders gesagt Lagerfähigkeit oder Laufzeit) von 3 Monaten bis zu 6 Monaten auf, mehr bevorzugt eine Stabilität von 3 bis zu 9 Monaten, mehr bevorzugt eine Stabilität von 3 bis zu 12 Monaten, mehr bevorzugt eine Stabilität von 3 bis zu 15 Monaten, mehr bevorzugt eine Stabilität von 3 bis zu 18 Monaten, mehr bevorzugt eine Stabilität von 3 bis zu 21 Monaten, mehr bevorzugt eine Stabilität von 3 bis zu 24 Monaten, mehr bevorzugt eine Stabilität von 3 bis zu 27 Monaten, mehr bevorzugt eine Stabilität von 3 bis zu 30 Monaten, mehr bevorzugt eine Stabilität von 3 bis zu 33 Monaten und noch mehr bevorzugt eine Stabilität von 3 bis zu 36 Monaten, wobei der Bortezomibgehalt bis zum Ende der Laufzeit 95 % des ursprünglich eingesetzten Bortezomibs nicht unterschreitet.

Die erfindungsgemäß hergestellte Lösung weist ferner erfindungsgemäß bevorzugt bei einer Temperatur von 25 °C eine Stabilität (oder anders gesagt Lagerfähigkeit oder Laufzeit) von zumindest 3 Monaten auf, bevorzugt von zumindest 6 Monaten, weiter bevorzugt von zumindest 12 Monaten und noch weiter bevorzugt von zumindest 24 Monaten, wobei der Bortezomibgehalt bis zum Ende der Laufzeit 90 %, bevorzugt 95 %, des ursprünglich eingesetzten Bortezomibs nicht unterschreitet.

Die vorliegende Erfindung betrifft ein Verfahren zur Herstellung einer gebrauchsfertigen flüssigen pharmazeutischen Bortezomibester-Lösung. Gebrauchsfertige Wirkstoff-haltige Lösungen sind im Stand der Technik bekannt und werden dort auch als *ready-to-use* (RTU)-Lösungen bezeichnet. Während als Lösung zu applizierende feste Wirkstoffformulierungen z.B. in Form von Pulvern oder Lyophilisaten in den Markt kommen und erst kurz vor ihrer Verabreichung mittels Rekonstitution in Lösung überführt werden, werden gebrauchsfertige Lösungen bereits in Form von Lösungen vermarktet.

Die erfindungsgemäß hergestellte Lösung kann beispielsweise unmittelbar, d.h. ohne weitere Zubereitung verabreicht werden oder auch erst nach einer weiteren Zubereitung wie etwa einem Verdünnen auf eine gewünschte Wirkstoffkonzentration.

Im Rahmen des erfindungsgemäßen Verfahrens wird die gebrauchsfertige Lösung mittels und auf Basis eines wässrigen Lösungsmittels hergestellt. Dabei wird unter einem wässrigen Lösungsmittel ein Lösungsmittel bzw. ein Lösungsmittelgemisch von bei einer Temperatur von 20 °C und einem Druck von 1013 mbar flüssigen Einzelkomponenten verstanden, das zumindest zu 50 Gew.-% aus Wasser besteht, bevorzugt zumindest zu 70 Gew.-% aus Wasser, mehr bevorzugt zumindest zu 90 Gew.-% aus Wasser und noch mehr bevorzugt zumindest zu 95 Gew.-% aus Wasser. In diesem Zusammenhang ist es erfindungsgemäß besonders bevorzugt, wenn das Lösungsmittel zumindest zu 99 Gew.-% aus Wasser besteht und noch weiter bevorzugt zu 100 Gew.-% aus Wasser.

Neben Wasser kann das Lösungsmittel ferner einen gewissen Anteil von einer oder mehreren mit Wasser mischbaren flüssigen organischen Komponenten enthalten. Als Beispiele für derartige organische Komponenten seien Ethanol und Isopropanol genannt.

In der erfindungsgemäß hergestellten Lösung ist ein Bortezomibester gelöst enthalten. Bortezomib ist eine sogenannte Boronsäure, die die funktionelle Gruppe -B(OH)2 enthält, welche mit einer eine oder mehrere Alkohol-Gruppen enthaltenden Bortezomibester-Bildungskomponente unter Abspaltung von Wasser und gegebenenfalls Säure verestert werden kann.

In dem erfindungsgemäßen Verfahren wird gemäß dem Schritt b) die Bortezomibester-Bildungskomponente in dem Lösungsmittel gelöst. Grundsätzlich kann der Schritt b) sowohl vor dem Schritt c) als auch nach dem Schritt c) durchgeführt werden. Gemäß einer bevorzugten Ausführungsform des erfindungsgemäßen Verfahrens ist es jedoch vorgesehen, dass der Schritt b) vor dem Schritt c) durchgeführt wird. Dadurch wird eine leichtere und verbesserte Löslichkeit des in Wasser nur verhältnismäßig schlecht löslichen Bortezomibs in dem Lösungsmittel gewährleistet.

Im Rahmen des erfindungsgemäßen Verfahrens wird gemäß dem Schritt d) der Sauerstoffgehalt des Lösungsmittels vermindert. Dabei wird in Schritt d) der Sauerstoffgehalt mit einer Maßnahme vermindert, die gezielt auf die Verminderung des Sauerstoffgehaltes im Lösungsmittel gerichtet ist und nicht etwa auf eine unkontrollierte Sauerstoffverminderung, wie sie beispielsweise beim unerwünschten oxidativen Bortezomibabbau auftritt.

Grundsätzlich kann der Schritt d) nach dem/den Schritt/Schritten a), b) und/oder c) durchgeführt werden. Gemäß einer bevorzugten Ausführungsform des erfindungsgemäßen Verfahrens ist es jedoch vorgesehen, dass der Schritt d) nach dem Schritt b) und vor dem Schritt c) durchgeführt wird. Durch diese Maßnahme kann mittels des erfindungsgemäßen Verfahrens auf verhältnismäßig kostengünstige Weise eine relativ stabile Bortezomibester-Lösung bereitgestellt werden.

Prinzipiell kann das Vermindern des Sauerstoffgehaltes des Lösungsmittels mit jeder Maßnahme durchgeführt werden, die für den zuständigen Fachmann im Hinblick auf die Durchführung des erfindungsgemäßen Verfahrens als geeignet erscheint, beispielsweise, indem das Lösungsmittel einem Vakuum unterworfen wird. Entsprechend einer bevorzugten Ausführungsform des erfindungsgemäßen Verfahrens hingegen ist es vorgesehen, dass der Schritt d) mittels Hindurchleiten eines Inertgases durch das Lösungsmittel durchgeführt wird. Dadurch kann das erfindungsgemäße Verfahren auf verfahrenstechnisch einfache und damit kostengünstige Weise durchgeführt werden. Als Inertgas kommen dabei erfindungsgemäß bevorzugt die Edelgase und Stickstoff in Betracht, wobei aus Kostengründen insbesondere Stickstoff bevorzugt ist.

Gemäß einer weiter bevorzugten Ausführungsform des erfindungsgemäßen Verfahrens ist es vorgesehen, dass der Sauerstoffgehalt des Lösungsmittels gemäß Schritt d) auf einen Wert von kleiner als 0,5 mg/l bis 0,01 mg/ml. Dadurch wird eine weitgehende Stabilität der mittels des erfindungsgemäßen Verfahrens erhältlichen Bortezomibester-Lösung gewährleistet. Der Sauerstoffgehalt in dem Lösungsmittel ist erfindungsgemäß mit einem Sauerstoff-Sensor basierend auf der Clark-Elektrode (US 2,913,386) gemäß Allen J., Lab Medicine 2003(34), 544-547)zu bestimmen.

In der erfindungsgemäß hergestellten Lösung ist ein Bortezomibmannitolester gelöst enthalten. Wie bereits vorstehend ausgeführt wurde, ist Bortezomib eine sogenannte Boronsäure, die die funktionelle Gruppe -B(OH)2 enthält, welche mit einer eine oder mehrere Alkohol-Gruppen enthaltenden Bortezomibester-Bildungskomponente unter Abspaltung von Wasser verestert werden kann. Der Offenbarung der vorliegenden Erfindung entsprechend können sämtliche Bortezomibester von Bortezomib mit einer Bortezomibester-Bildungskomponente eingesetzt werden, die für den erfindungsgemäßen pharmazeutischen Zweck geeignet sind. Bevorzugt ist es jedoch, wenn der Bortezomibester ein Ester des Bortezomibs mit einem Polyol als Bortezomibester-Bildungskomponente ist. Dadurch wird eine verhältnismäßig hohe Stabilität der mittels des offenbarten Verfahrens erhältlichen gebrauchsfertigen Bortezomibester-Lösung gewährleistet. Bevorzugt sind dabei insbesondere solche Bortezomib-Polyolester, in denen die beiden OH-Gruppen der Gruppe -B(OH)2 eines Bortezomib-Moleküls mit zwei OH-Gruppen eines Polyol-Moleküls unter Abspaltung von Wasser und Ausbildung einer Ringstruktur verestert sind, wie zum Beispiel in EP 1 355 910 anhand von Strukturformeln gezeigt.

Gemäß einer weiter bevorzugten Ausführungsform des offenbarten Verfahrens ist es vorgesehen, dass die Bortezomibester-Bildungskomponente ein Alditol ist. Eine mittels des offenbarten Verfahrens hergestellte Bortezomib-Alditolester-Lösung ist durch eine verhältnismäßig hohe Stabilität gekennzeichnet. Bevorzugt sind dabei insbesondere solche Bortezomib-Alditolester, in denen die beiden OH-Gruppen der Gruppe -B(OH)2 eines Bortezomib-Moleküls mit zwei OH-Gruppen eines Alditol-Moleküls unter Abspaltung von Wasser und Ausbildung einer Ringstruktur verestert sind, wie zum Beispiel in EP 1 355 910 anhand von Strukturformeln gezeigt.

Alditole sind im Stand der Technik bekannt und entsprechen der nachstehenden allgemeinen Strukturformel, wobei n aus den natürlichen Zahlen ausgewählt und größer/gleich 1 ist

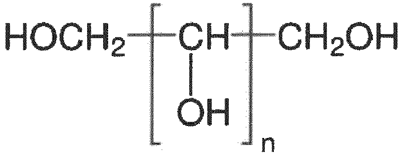

Entsprechend einer weiter bevorzugten Ausführungsform des offenbarten Verfahrens ist es vorgesehen, dass die Bortezomibester-Bildungskomponente ein Alditol der voranstehenden Strukturformel mit n=1 bis 10 ist, vorzugsweise ein Alditol mit n=2 bis 4. Mittels des offenbarten Verfahrens erhältliche Lösungen enthaltend einen Bortezomib-Alditolester besagter Auswahl zeichnen sich durch eine verhältnismäßig hohe Stabilität und hohe Löslichkeit aus.

Nach einer weiter bevorzugten Ausführungsform des offenbarten Verfahrens ist es vorgesehen, dass die Bortezomibester-Bildungskomponente ausgewählt ist aus der Gruppe bestehend aus Arabitol, Dulcitol, Erythritol, Mannitol, Ribitol, Sorbitol und Xylitol und insbesondere aus der Gruppe bestehend aus Mannitol, Sorbitol und Xylitol, wobei Mannitol erfindungsgemäß und dabei insbesondere D-Mannitol besonders bevorzugt ist. Unter Durchführung des offenbarten Verfahrens erhältliche Lösungen enthaltend einen Bortezomib-Alditolester besagter Alditole zeichnen sich durch eine besonders hohe Stabilität aus.

In der mittels des erfindungsgemäßen Verfahrens erhältlichen Lösung liegt der Bortezomibester im Gleichgewicht mit seinen Hydrolyseprodukten freies Bortezomib und freie Bortezomibester-Bildungskomponente vor. Um die Stabilität der mittels des erfindungsgemäßen Verfahrens erhältlichen Lösung weiter zu erhöhen, ist es entsprechend einer weiter bevorzugten Ausführungsform der Erfindung vorgesehen, dass die Bortezomibester-Bildungskomponente in Bezug auf Bortezomib in einem zumindest 10-fachen molaren Überschuss eingesetzt wird, bevorzugt in einem 10 bis 400-fachen molaren Überschuss und besonders bevorzugt in einem 10 bis 100-fachen molaren Überschuss und ganz besonders bevorzugt in einem 15 bis 30-fachen molaren Überschuss.

Gemäß einer weiter bevorzugten Ausführungsform des erfindungsgemäßen Verfahrens ist es vorgesehen, dass zumindest 90 % des in der resultierenden Lösung enthaltenen Bortezomibs in Form eines Esters vorliegen, weiter bevorzugt zumindest 95 % und noch mehr bevorzugt zumindest 99 %. Dadurch wird eine verhältnismäßig hohe Stabilität der mittels des erfindungsgemäßen Verfahrens erhältlichen Lösungen gewährleistet. Der Grad der Veresterung des Bortezomibs in der resultierenden Lösung kann durch dem Fachmann bekannte Maßnahmen eingestellt werden, wie z.B. durch den pH-Wert der Lösung oder über den Gehalt der Lösung an freier Bortezomibester-Bildungskomponente. Der Grad der Veresterung des Bortezomibs wird erfindungsgemäß bevorzugt durch eine Titration und des ermittelten apparenten pka-Wertes des Bortezomibs oder durch ¹¹Bor-NMR bestimmt (siehe W. Marinaro, Physical and chemical properties of boronic acids: Formulation implications, Dissertation University Kansas, ProQuest, UMI Dissertations Publishing 2007, ISBN-13: 978-0-549-13739-9).

Entsprechend einer weiter bevorzugten Ausführungsform des erfindungsgemäßen Verfahrens ist es vorgesehen, dass in dem Lösungsmittel Bortezomib in einer Menge von 0,5 mg bis 10 mg pro Milliliter Lösungsmittel gelöst wird, bevorzugt in einer Menge von 1,0 mg/ml bis 5 mg/ml.

Nach einer weiter bevorzugten Ausführungsform des erfindungsgemäßen Verfahrens ist es vorgesehen, dass in dem Lösungsmittel ferner NaCl gelöst wird. Es konnte gezeigt werden, dass NaCl die Stabilität der mittels des erfindungsgemäßen Verfahrens erhältlichen Bortezomibester-Lösung weiter verbessert.

Gemäß einer weiter bevorzugten Ausführungsform des erfindungsgemäßen Verfahrens ist es vorgesehen, dass das Lösungsmittel auf einen pH-Wert (gemessen bei einer Temperatur von 20 °C) in einem Bereich von 3 bis 6 eingestellt wird, bevorzugt auf einen pH-Wert in einem Bereich von 3 bis 5 und besonders bevorzugt auf einen pH-Wert in einem Bereich von 3,5 bis 4,5. Es konnte gezeigt werden, dass eine mittels des erfindungsgemäßen Verfahrens erhältliche Lösung in den genannten pH-Wert-Bereichen durch eine besonders hohe Stabilität gekennzeichnet ist.

Entsprechend einer weiter bevorzugten Ausführungsform des erfindungsgemäßen Verfahrens ist es vorgesehen, dass die Einstellung des pH-Wertes nach Schritt c) erfolgt.

In einer weiter bevorzugten Ausführungsform des erfindungsgemäßen Verfahrens ist es vorgesehen, zur Einstellung des pH-Werts der mittels des erfindungsgemäßen Verfahrens erhältlichen Lösung Salzsäure oder Natriumhydroxid zu verwenden, je nachdem, welcher pH-Wert eingestellt werden soll.

Um den pH-Wert der mittels des erfindungsgemäßen Verfahrens erhältlichen Lösung in einem pH-Wert Bereich von 3,7 bis 5,7 möglichst konstant zu halten und damit einhergehend eine erhöhte Stabilität der Lösung dauerhaft zu gewährleisten, ist es entsprechend einer weiter bevorzugten Ausführungsform der Erfindung vorgesehen, dass in dem Lösungsmittel ein Essigsäure/Acetat-Puffersystem gelöst wird.

Um den pH-Wert der mittels des erfindungsgemäßen Verfahrens erhältlichen Lösung in einem pH-Wert Bereich von 2,8 bis 4,8 möglichst konstant zu halten und damit einhergehend eine erhöhte Stabilität der Lösung dauerhaft zu gewährleisten, ist es entsprechend einer weiter bevorzugten Ausführungsform der Erfindung vorgesehen, dass in der Lösung ein Milchsäure/Lactat-Puffersystem gelöst ist.

Gemäß einer weiter bevorzugten Ausführungsform des erfindungsgemäßen Verfahrens ist es vorgesehen, dass die resultierende Bortezomibester-Lösung derart ausgebildet wird, dass sie zur parenteralen Verabreichung geeignet ist, vorzugsweise zur intravenösen und/oder subkutanen Verabreichung.

Nach einer weiter bevorzugten Ausführungsform des erfindungsgemäßen Verfahrens ist es vorgesehen, dass die resultierende Bortezomibester-Lösung frei von Stabilisatoren ist, insbesondere frei von Chelatoren und Antioxidantien. Überraschenderweise wurde festgestellt, dass die resultierende Lösung trotz Abwesenheit von Chelatoren wie Ethylendiamintetraessigsäure (EDTA) und Antioxidantien ausreichend stabil ist.

Um die physiologische Toleranz der mittels des erfindungsgemäßen Verfahrens erhältlichen Lösung weiter zu verbessern, ist es entsprechend einer weiter bevorzugten Ausführungsform der Erfindung vorgesehen, dass das Lösungsmittel frei von einem organischen Lösungsmittel eingesetzt wird. Entsprechend ist dann auch die aus dem erfindungsgemäßen Verfahren resultierende Bortezomibester-Lösung frei von einem organischen Lösungsmittel.

Gemäß einer weiter bevorzugten Ausführungsform des erfindungsgemäßen Verfahrens ist es vorgesehen, dass die resultierende Bortezomibester-Lösung frei von Liposomen und/oder frei von Detergentien ist. Diese Maßnahme trägt ebenfalls zur Verbesserung der physiologischen Toleranz der mittels des erfindungsgemäßen Verfahrens erhältlichen Lösung bei.

Nach einer weiter bevorzugten Ausführungsform des erfindungsgemäßen Verfahrens ist es vorgesehen, dass die mittels des erfindungsgemäßen Verfahrens erhältliche Bortezomibester-Lösung steril ist.

Entsprechend einer weiter bevorzugten Ausführungsform des erfindungsgemäßen Verfahrens ist es vorgesehen, dass nach dem Schritt d) des Hindurchleitens des Inertgases durch das Lösungsmittel die Bortezomibester-Lösung einem Vakuum ausgesetzt wird.

Gemäß einer weiter bevorzugten Ausführungsform des erfindungsgemäßen Verfahrens ist es vorgesehen, dass das Verfahren ferner den Schritt des Abfüllens der Bortezomibester-Lösung in einen Behälter sowie ein luftdichtes Verschließen des befüllten Behälters umfasst.

Nach einer weiter bevorzugten Ausführungsform des erfindungsgemäßen Verfahrens ist es vorgesehen, dass das Abfüllen der Bortezomibester-Lösung in den Behälter in einer Inertgasatmosphäre erfolgt. Dadurch wird ein Kontakt zwischen der mittels des erfindungsgemäßen Verfahrens erhältlichen Lösung sowie dem Luftsauerstoff weitgehend vermieden, was die Stabilität der Bortezomibester-Lösung positiv beeinflusst.

Entsprechend einer weiter bevorzugten Ausführungsform des erfindungsgemäßen Verfahrens ist es vorgesehen, dass der Schritt d) nach dem Schritt b) und vor dem Schritt c) durchgeführt wird sowie nochmals nach dem Schritt c) und vor dem Abfüllen der Bortezomibester-Lösung in den Behälter, was sich ebenfalls positiv auf die Stabilität der Bortezomibester-Lösung auswirkt.

Gemäß einer weiter bevorzugten Ausführungsform des erfindungsgemäßen Verfahrens ist es vorgesehen, dass der Behälter aus einem Material gebildet ist, das für Sauerstoff nicht permeabel ist.

Nach einer weiter bevorzugten Ausführungsform des erfindungsgemäßen Verfahrens ist es vorgesehen, dass der Behälter aus Glas gebildet ist.

Entsprechend einer weiter bevorzugten Ausführungsform des erfindungsgemäßen Verfahrens ist es vorgesehen, dass der Behälter ein Vial ist.

Gemäß einer weiter bevorzugten Ausführungsform des erfindungsgemäßen Verfahrens ist es vorgesehen, dass der Kopfraum des Behälters mit einem Inertgas befüllt ist. Der Kopfraum des Behälters ist der Raum des Innenraums des Behälters, der nicht von der Bortezomibester-Lösung eingenommen wird. Als Inertgas kommen beispielsweise die Edelgase und Stickstoff in Betracht, wobei aus Kostengründen insbesondere Stickstoff bevorzugt ist.

Nach einer weiter bevorzugten Ausführungsform des erfindungsgemäßen Verfahrens ist es vorgesehen, dass im Kopfraum des Behälters ein Druck in einem Bereich von 870 mbar bis 1085 mbar eingestellt wird, wobei ein Druck von 950 mbar bis 1050 mbar, ein Druck von 1010 mbar bis 1050 mbar oder ein Druck von 1020 mbar bis 1030 mbar besonders bevorzugt ist.

Entsprechend einer weiter bevorzugten Ausführungsform des erfindungsgemäßen Verfahrens ist es vorgesehen, dass in dem Kopfraum des luftdicht verschlossenen Behälters ein Sauerstoffgehalt von maximal 0,15 Vol.-% eingestellt wird, vorzugsweise ein Sauerstoffgehalt von 0,15 Vol.-% bis 0,005 Vol.-%. Der Sauerstoffgehalt im Kopfraum ist erfindungsgemäß bevorzugt mittels Gaschromatographie unter Verwendung eines Flammenionisationsdetektors oder mittels Nahinfrarot-Dioden-Lasermessung gemäß Posset et al., Pharm Ind. 77(5), 739-747 (2015), zu bestimmen.

Entsprechend einer weiter bevorzugten Ausführungsform des erfindungsgemäßen Verfahrens ist es vorgesehen, dass das Verhältnis von Kopfraum zu Innenraum des abgedichteten Behältnisses einen Wert von kleiner als 0,95 aufweist, bevorzugt auf einen Wert von 0,75 bis 0,95. In einer weiteren bevorzugten Ausführungsform ist es vorgesehen, dass das Verhältnis von Kopfraum zu Innenraum des abgedichteten Behältnisses einen Wert von kleiner als 0,75 aufweist, bevorzugt einen Wert von 0,5 bis 0,75.

Es konnte festgestellt werden, dass der in der erfindungsgemäß herzustellenden Lösung enthaltene Wirkstoff einem durch Lichteinwirkung bedingten Abbau unterliegt. Entsprechend einer weiter bevorzugten Ausführungsform des erfindungsgemäßen Verfahrens ist es daher vorgesehen, dass das Verfahren weitestgehend, und besonders bevorzugt vollständig, unter Ausschluss von Lichteinwirkung durchgeführt wird.

Eine typische, mittels des erfindungsgemäßen Verfahrens erhältliche Bortezomibester-Lösung enthält 65 Gew.-% bis 99,85 Gew.-% Wasser, 0,05 Gew.-% bis 1 Gew.-% Bortezomib (in freier und veresterter Form berechnet als freies Bortezomib), 0,1 bis 20 Gew.-% Mannitol (in freier und veresterter Form berechnet als freies Mannitol) sowie 0 Gew.-% bis 14 Gew.-% pharmazeutische Hilfsstoffe, beispielsweise NaCl und/oder ein organisches Lösungsmittel.

Eine weitere typische, mittels des erfindungsgemäßen Verfahrens erhältliche Lösung enthält 80 Gew.-% bis 99,85 Gew.-% Wasser, 0,05 Gew.-% bis 1 Gew.-% Bortezomib (in freier und veresterter Form berechnet als freies Bortezomib), 0,1 bis 5 Gew.-% Mannitol (in freier und veresterter Form berechnet als freies Mannitol) sowie 0 Gew.-% bis 14 Gew.-% pharmazeutische Hilfsstoffe, beispielsweise NaCl und/oder ein organisches Lösungsmittel.

Vorliegend wird ferner eine gebrauchsfertige flüssige pharmazeutische Bortezomibester-Lösung offenbart, die gemäß dem erfindungsgemäßen Verfahren erhältlich ist.

Es konnte festgestellt werden, dass der in der Lösung enthaltene Wirkstoff einem durch Lichteinwirkung bedingten Abbau unterliegt. Die mit dem erfindungsgemäßen Verfahren erhältliche Lösung wird daher bevorzugt in einem Behälter aufbewahrt, wobei der Behälter die Lösung vor Lichteinwirkung schützt oder der Behälter die Lichteinwirkung auf die Lösung vermindert. Dabei kann der Behälter beispielsweise ein Primärverpackungsbehältnis - wie etwa ein Ampulle aus Braunglas - sein oder ein Sekundärverpackungsbehältnis - wie etwa eine handelsübliche Faltschachtel als Umverpackung.

Die mittels des erfindungsgemäßen Verfahrens erhältliche Bortezomibester-Lösung kann zur Behandlung des multiplen Myeloms und/oder des Mantelzelllymphoms verwendet werden.

Die nachstehende Beschreibung einiger bevorzugter Ausführungsformen der Erfindung dient deren Erläuterung.

Das erfindungsgemäße Verfahren kann beispielsweise unter Zuhilfenahme der Offenbarung der US 6,274,169 durchgeführt werden, insbesondere unter Zuhilfenahme der dort abgebildeten und beschriebenen Vorrichtungen.

### Ausführungsbeispiel 1

1250,0 g Mannitol wurden in 48,88 kg Wasser unter Rühren gelöst. Durch die so erhaltene Lösung wurde unter Rühren solange sterilfiltrierter Stickstoff hindurch geleitet, bis der Sauerstoffgehalt der Lösung 0,5 mg/l betrug (gemessen mittels Clark-Elektrode, Mettler Toledo InPro 6800). Die so hergestellte Mannitol-Lösung wurde dann auf eine Temperatur von 45 °C erwärmt. In der erwärmten Mannitol-Lösung wurden anschließend zunächst 125,0 g Bortezomib und dann 450,0 g NaCl unter Rühren gelöst.

Die wie vorstehend ausgeführt erhaltene Bortezomib-Mannitol-Lösung wurde nach dem Lösen des NaCl zunächst mit raumtemperiertem Wasser auf ein finales Volumen von 50 l aufgefüllt und dann auf eine Temperatur von 20 °C abgekühlt. Anschließend wurde der pH der abgekühlten Lösung mittels 1 %iger wässriger NaOH- bzw. HCl-Lösung auf einen Wert von 4,3 eingestellt. Nach der pH-Wert-Einstellung wurde durch die Bortezomib-Mannitol-Lösung unter Rühren nochmals solange sterilfiltrierter Stickstoff hindurch geleitet, bis der Sauerstoffgehalt der Lösung 0,5 mg/l betrug (gemessen mittels Clark-Elektrode, Mettler Toledo InPro 6800). Danach wurde die Bortezomib-Mannitol-Lösung mittels einer Sterilfiltrationskapsel unter Anwendung eines Stickstoffdrucks von 1,5 bar sterilfiltriert.

Die vorgenannte sterilfiltrierte Lösung wurde unter einer Stickstoffatmosphäre in mehrfach mit Stickstoff gespülte Vials (10R DIN, 13 mm Hals) mit einem Volumen von (12,15 ml) zu Portionen von 1,4 ml abgefüllt. Die befüllten offenen Vials wurden anschließend in eine Lyophilisierungskammer verbracht. Die Kammer wurde bei einer Temperatur von 20 °C auf einen Druck von 50 mbar +/- 10 mbar evakuiert und anschließend mit sterilfiltriertem Stickstoff geflutet, wobei ein Stickstoffdruck von 950 +/- 25 mbar eingestellt wurde. Besagter Evakuierungs/Flut-Vorgang wurde anschließend noch zweimal wiederholt, wobei bei der zweiten Wiederholung beim Fluten mit Stickstoff der Stickstoffdruck in der Kammer auf einen Wert von 1013 mbar eingestellt wurde. Die Vials wurden in der Kammer unter Stickstoffatmosphäre mit einem Gummistopfen verschlossen, der Kammer entnommen und dann mit einer Aluminiumkappe verbördelt. Der Sauerstoffgehalt im Kopfraum des Vials betrug unmittelbar nach dem Verschließen und Verbördeln 0,15 Vol.-% (gemessen mittels Gaschromatographie und einem Flammenionisationsdetektor: Probenzug: Verdrängung des Gases im Vialkopfraum durch eingepresstes Wasser bei gleichzeitiger Entnahme über ein Dreiwegeventil; das Vial wird hierzu *bottom up* unter Wasser getaucht in einer Fixierung eingespannt; Bestimmung von Sauerstoff neben Stickstoff; Säule: Molekularsieb 5 A, 50 m, 0,53 *µ*m; Trägergas: Helium (ca. 80 kPa); Injektion: manuell; Bemerkung: da der Sauerstoffgehalt in der Probe deutlich kleiner als die Bestimmungsgrenze (Ref.-Stdd. 0,50 % (Vol./Vol.) Sauerstoff) ist, kann über einen Einpunktkalibrierung-Limittest am LOQ (0,1 % (Vol./Vol.)) ausgewertet werden).

### Stabilitätsuntersuchung

Befüllte Vials gemäß Ausführungsbeispiel 1 wurden über einen Zeitraum von 6 Monaten bei Temperaturen von 2-8 °C, 15 °C und 25 °C eingelagert. Nach einem, zwei, drei und sechs Monat/en wurde der Bortezomibgehalt der Lösungen mittels *high-performance liquid chromatography* (HPLC) wie weiter unten ausgeführt bestimmt. In der nachstehenden Tabelle sind die relativen Bortezomibgehalte der eingelagerten Lösungen in Bezug auf ursprünglich eingesetztes Bortezomib in Prozent angegeben.

| Zeit | 2-8 °C | 15 °C | 25 °C |
|---|---|---|---|
| 1 Monat | - | - | 101,4 |
| 2 Monate | 101,8 | 101,5 | 100,9 |
| 3 Monate | 102,3 | 102,1 | 100,7 |
| 6 Monate | 101,1 | 100,2 | 97, 9 |

### Ausführungsbeispiel 2

Es wurden Vials analog Ausführungsbeispiel 1 präpariert, mit dem einzigen Unterschied, dass der pH der Lösung mit 1N Salzsäure und 1N Natronlauge auf einen Wert von 3,0, 3,5, 4,0, 4,5, 5,0, 5,5, 6,0 bzw. 6,5 eingestellt wurde.

### Stabilitätsuntersuchung

Befüllte Vials gemäß Ausführungsbeispiel 2 wurden über einen Zeitraum von 4 Wochen bei einer Temperatur von 40 °C eingelagert. Nach einer, zwei, drei und vier Woche/n der Einlagerung wurde der Bortezomibgehalt der Lösungen mittels HPLC wie weiter unten ausgeführt bestimmt.

Die Auswertung der Bortezomibgehalte der Lösungen ergab, dass eine Lösung die höchste Stabilität aufweist, wenn der pH-Wert der Lösung auf einen Wert von 3,5 bis 4,5 eingestellt wurde.

### HPLC

Der relative Gehalt der Lösungen an Bortezomib bezogen auf die ursprüngliche Einwaage wurde mittels HPLC gemäß Europäischem Arzneibuch, 8. Ausgabe, Ziffer 2.2.29, Seiten 60 bis 62 mit den folgenden Systemparametern bestimmt:
- Gerät: HPLC SpectraSYSTEM von Thermo Electron Corp. ausgestattet mit Diodenfelddetektor sowie einen Säulenofen;
- Säule: SymmetryShield RP 18, 5 *µ*m, 250 x 4,6 mm, Waters Artikelnummer 186000112;
- mobile Phase A: entgaste Mischung von 715 ml Wasser, 285 ml Acetonitril und 1 ml Ameisensäure;
- mobile Phase B: entgaste Mischung von 200 ml Wasser, 800 ml Methanol und 1 ml Ameisensäure;
- Flussrate: 1,0 ml/min;
- Injektionsvolumen: 20 *µ*l;
- Detektion: Diodenfeldspektralphotometer mit einer bei 270 nm festgelegten Wellenlänge und einer spektralen Abtastung von 200 bis 420 nm;
- Temperatur: 35 °C;
- externer Standard: 10 mg Bortezomib gelöst in 20 ml Diluent
- Probenvorbereitung: 1 ml einer Bortezomib-Lösung mit einer Konzentration von 2,5 mg/ml werden mit Diluent auf ein Volumen von 5 ml verdünnt
- Diluent: Wasser / Acetonitril 70:30 (Vol.-%/Vol.-%)
- Gradient:

| Zeit [min] | mobile Phase A [Vol. -%] | mobile Phase A [Vol.-%] |
|---|---|---|
| 0 - 20 | 100 | 0 |
| 20 - 35 | 100 → 0 | 0 → 100 |
| 35 - 50 | 0 | 100 |
| 50 - 52 | 0 →100 | 100 → 0 |
| 52 - 57 | 100 | 0 |

## Patentansprüche

1. Verfahren zur Herstellung einer gebrauchsfertigen flüssigen pharmazeutischen Bortezomibester-Lösung, umfassend die Schritte des
a) Bereitstellens eines wässrigen Lösungsmittels;
b) Lösens einer Bortezomibester-Bildungskomponente in dem Lösungsmittel, wobei als Bortezomibester-Bildungskomponente Mannitol eingesetzt wird;
c) Lösens von Bortezomib oder einem pharmazeutisch annehmbaren Salz davon in dem Lösungsmittel;
d) Verminderns des Sauerstoffgehaltes des Lösungsmittels auf einen Wert von kleiner als 0,5 mg/l.

2. Verfahren nach Anspruch 1, **dadurch gekennzeichnet, dass** der Schritt b) vor dem Schritt c) durchgeführt wird.

3. Verfahren nach einem der voranstehenden Ansprüche, **dadurch gekennzeichnet, dass** der Schritt d) nach dem Schritt b) und vor dem Schritt c) durchgeführt wird.

4. Verfahren nach einem der voranstehenden Ansprüche, **dadurch gekennzeichnet, dass** der Schritt d) mittels Hindurchleiten eines Inertgases durch das Lösungsmittel durchgeführt wird.

5. Verfahren nach einem der voranstehenden Ansprüche, **dadurch gekennzeichnet, dass** der Sauerstoffgehalt des Lösungsmittels gemäß Schritt d) auf einen Wert von kleiner als 0,5 mg/l bis 0,01 mg/ml vermindert wird.

6. Verfahren nach einem der voranstehenden Ansprüche, **dadurch gekennzeichnet, dass** die Bortezomibester-Bildungskomponente in Bezug auf Bortezomib in einem zumindest 10-fachen molaren Überschuss eingesetzt wird, bevorzugt in einem 10 bis 400-fachen molaren Überschuss und besonders bevorzugt in einem 10 bis 100-fachen molaren Überschuss und ganz besonders bevorzugt in einem 15 bis 30-fachen molaren Überschuss.

7. Verfahren nach einem der voranstehenden Ansprüche, **dadurch gekennzeichnet, dass** in dem Lösungsmittel Bortezomib in einer Menge von 0,5 mg bis 10 mg pro Milliliter Lösungsmittel gelöst wird, bevorzugt in einer Menge von 1,0 mg/ml bis 5 mg/ml.

8. Verfahren nach einem der voranstehenden Ansprüche, **dadurch gekennzeichnet, dass** das Lösungsmittel auf einen pH-Wert in einem Bereich von 3 bis 6 eingestellt wird, bevorzugt auf einen pH-Wert in einem Bereich von 3 bis 5 und besonders bevorzugt auf einen pH-Wert in einem Bereich von 3,5 bis 4,5.

9. Verfahren nach Anspruch 8, **dadurch gekennzeichnet, dass** die Einstellung des pH-Wertes nach dem Schritt c) erfolgt.

10. Verfahren nach Anspruch 4, **dadurch gekennzeichnet, dass** nach dem Schritt d) des Hindurchleitens des Inertgases durch das Lösungsmittel die Bortezomibester-Lösung einem Vakuum ausgesetzt wird.

11. Verfahren nach einem der voranstehenden Ansprüche, **dadurch gekennzeichnet, dass** das Verfahren ferner den Schritt des Abfüllens der Bortezomibester-Lösung in einen Behälter sowie ein luftdichtes Verschließen des befüllten Behälters umfasst.

12. Verfahren nach Anspruch 11, **dadurch gekennzeichnet, dass** das Abfüllen der Bortezomibester-Lösung in den Behälter in einer Inertgasatmosphäre erfolgt.

13. Verfahren nach Anspruch 11 oder 12, **dadurch gekennzeichnet, dass** der Schritt d) nach dem Schritt b) und vor dem Schritt c) durchgeführt wird sowie nochmals nach dem Schritt c) und vor dem Abfüllen der Bortezomibester-Lösung in den Behälter.

## Claims

1. Process for producing a ready-to-use liquid pharmaceutical bortezomib ester solution, comprising the steps of
a) providing an aqueous solvent;
b) dissolving a component for forming a bortezomib ester in the solvent, the component used for forming a bortezomib ester being mannitol;
c) dissolving bortezomib or a pharmaceutically acceptable salt thereof in the solvent;
d) reducing the oxygen content of the solvent to a value of less than 0.5 mg/l.

2. Process according to Claim 1, **characterized in that** step b) is carried out before step c).

3. Process according to either of the preceding claims, **characterized in that** step d) is carried out after step b) and before step c).

4. Process according to any of the preceding claims, **characterized in that** step d) is carried out by passing an inert gas through the solvent.

5. Process according to any of the preceding claims, **characterized in that** the oxygen content of the solvent is according to step d) reduced to a value of less than 0.5 mg/l to 0.01 mg/ml.

6. Process according to any of the preceding claims, **characterized in that** the component for forming a bortezomib ester is, based on bortezomib, used in an at least 10 times molar excess, preferably in a 10 to 400 times molar excess, particularly preferably in a 10 to 100 times molar excess and very particularly preferably in a 15 to 30 times molar excess.

7. Process according to any of the preceding claims, **characterized in that** bortezomib is dissolved in the solvent in an amount of 0.5 mg to 10 mg per millilitre of solvent, preferably in an amount of 1.0 mg/ml to 5 mg/ml.

8. Process according to any of the preceding claims, **characterized in that** the solvent is adjusted to a pH within a range from 3 to 6, preferably to a pH within a range from 3 to 5 and more preferably to a pH within a range from 3.5 to 4.5.

9. Process according to Claim 8, **characterized in that** the adjustment of the pH takes place after step c).

10. Process according to Claim 4, **characterized in that**, after step d) of passing the inert gas through the solvent, the bortezomib ester solution is exposed to a reduced pressure.

11. Process according to any of the preceding claims, **characterized in that** the process additionally includes the step of transferring the bortezomib ester solution to a container and closing the filled container in an airtight manner.

12. Process according to Claim 11, **characterized in that** the transfer of the bortezomib ester solution to the container takes place in an inert gas atmosphere.

13. Process according to Claim 11 or 12, **characterized in that** step d) is carried out after step b) and before step c) and also one more time after step c) and before transfer of the bortezomib ester solution to the container.

## Revendications

1. Procédé pour produire une solution pharmaceutique liquide prête à l'emploi d'ester de bortézomib, comprenant les étapes consistant à :
a) fournir un solvant aqueux ;
b) dissoudre un composant de formation d'ester de bortézomib dans le solvant, en utilisant du mannitol comme composant de formation d'ester de bortézomib ;
c) dissoudre le bortézomib ou un sel pharmaceutiquement acceptable de celui-ci dans le solvant ;
d) réduire la teneur en oxygène du solvant à une valeur inférieure à 0,5 mg/l.

2. Procédé selon la revendication 1, **caractérisé en ce que** l'étape d) est réalisée avant l'étape c).

3. Procédé selon l'une quelconque des revendications précédentes, **caractérisé en ce que** l'étape d) est réalisée après l'étape b) et avant l'étape c).

4. Procédé selon l'une quelconque des revendications précédentes, **caractérisé en ce que** l'étape d) est réalisée en faisant passer un gaz inerte à travers le solvant.

5. Procédé selon l'une quelconque des revendications précédentes, **caractérisé en ce que** la teneur en oxygène du solvant selon l'étape d) est réduite d'une valeur inférieure à 0,5 mg/1 à 0,01 mg/ml.

6. Procédé selon l'une quelconque des revendications précédentes, **caractérisé en ce que** le composant de formation d'ester de bortézomib est utilisé par rapport au bortézomib à un excès molaire d'au moins 10 fois, de préférence à un excès molaire de 10 à 400 fois et de manière particulièrement préférée à un excès molaire de 10 à 100 fois et de manière plus particulièrement préférée à un excès molaire de 15 à 30 fois.

7. Procédé selon l'une quelconque des revendications précédentes, **caractérisé en ce que** le bortézomib est dissous dans le solvant en une quantité de 0,5 mg à 10 mg par millilitre de solvant, de préférence en une quantité de 1,0 mg/ml à 5 mg/ml.

8. Procédé selon l'une quelconque des revendications précédentes, **caractérisé en ce que** le solvant est ajusté à un pH compris entre 3 et 6, de préférence à un pH compris entre 3 et 5, et de manière particulièrement préférée à un pH compris entre 3,5 et 4,5.

9. Procédé selon la revendication 8, **caractérisé en ce que** l'ajustement de la valeur de pH survient après l'étape c).

10. Procédé selon la revendication 4, **caractérisé en ce qu'**après l'étape d) de passage du gaz inerte à travers le solvant, la solution d'ester de bortézomib est soumise à un vide.

11. Procédé selon l'une quelconque des revendications précédentes, **caractérisé en ce que** le procédé comprend en outre l'étape de remplissage d'un récipient avec la solution d'ester de bortézomib et de fermeture du récipient rempli d'une manière étanche à l'air.

12. Procédé selon la revendication 11, **caractérisé en ce que** le remplissage du récipient avec la solution d'ester de bortézomib survient dans une atmosphère de gaz inerte.

13. Procédé selon la revendication 11 ou 12, **caractérisé en ce que** l'étape d) est réalisée après l'étape b) et avant l'étape c), et de nouveau après l'étape c) et avant le remplissage du récipient avec la solution d'ester de bortézomib.
